(19) Europäisches Patentamt
European Patent Office
Office européen des brevets

(11) **EP 1 649 255 B1**

(12) **EUROPEAN PATENT SPECIFICATION**

(45) Date of publication and mention
of the grant of the patent:
**07.02.2007 Bulletin 2007/06**

(51) Int Cl.:
*G01J 3/50* (2006.01)    *G01N 21/47* (2006.01)
*G01J 3/51* (2006.01)    *G02B 21/08* (2006.01)

(21) Application number: **03817818.2**

(22) Date of filing: **01.08.2003**

(86) International application number:
**PCT/TR2003/000064**

(87) International publication number:
**WO 2005/012858 (10.02.2005 Gazette 2005/06)**

(54) **DEVICE FOR MEASURING COLOR AND COLOR DIFFERENCES AND A COLOUR MEASURING METHOD REALIZED WITH THIS DEVICE**

EINRICHTUNG ZUM MESSEN VON FARBEN UND FARBDIFFERENZEN UND FARBMESSVERFAHREN, DAS MIT DIESER EINRICHTUNG REALISIERT WIRD

DISPOSITIF DE MESURE DE COULEUR ET DE DIFFERENCES DE COULEUR, ET PROCEDE ASSOCIE MIS EN OEUVRE AU MOYEN DE CE DISPOSITIF

(84) Designated Contracting States:
**DE GB TR**

(43) Date of publication of application:
**26.04.2006 Bulletin 2006/17**

(73) Proprietor: **Tubitak Uekae**
**41470 Kocaeli (TK)**

(72) Inventors:
• **HACIZADE, Fikret**
**41470 Kocaeli (TR)**
• **MAMEDBEYLI, Izmir Keyan**
**Kartal,**
**Istanbul (TR)**

(74) Representative: **Cayli, Hülya**
**Paragon Consultancy Inc.**
**Koza Sokak No: 60/6**
**GOP**
**06700 Ankara (TR)**

(56) References cited:
**EP-A- 0 570 003**    **WO-A-02/057752**
**DE-A- 19 615 971**    **DE-C- 834 769**
**JP-A- 61 041 362**    **US-A1- 2002 071 124**

• **PATENT ABSTRACTS OF JAPAN vol. 0164, no. 18 (P-1413), 3 September 1992 (1992-09-03) & JP 4 140620 A (SHIMADZU CORP), 14 May 1992 (1992-05-14)**
• **PATENT ABSTRACTS OF JAPAN vol. 1998, no. 12, 31 October 1998 (1998-10-31) & JP 10 185812 A (SUMITOMO METAL IND LTD), 14 July 1998 (1998-07-14)**
• **PATENT ABSTRACTS OF JAPAN vol. 0061, no. 68 (P-139), 2 September 1982 (1982-09-02) & JP 57 085176 A (NIPPON ELECTRIC IND CO LTD), 27 May 1982 (1982-05-27)**

**Description**

**Related Technical Field**

This invention is related with the measurement of color and color differences in all kinds of monochrome or colorful textured material (i.e. jean) either in thread or particle form.

**Prior Art**

Generally the procedures of the International Commission on Illumination - CIE are used in the measurement of color and color differences. The aim is to standardize and regulate the end products of different devices which digitize human perception and which are produced by different manufacturers.

In the procedure of color and color differences measurement, the most important function is to determine the reflection coefficient **R(λ)** of the specimen in the area of the electromagnetic spectrum in which the human eye is sensitive (between 380 - 780 nm wavelengths). In order to realize this operation a lighting device with a light source of 380-780 nm wavelengths (polychromatic light) and a receptive unit which differentiates the spectral value of the light projected and records the signal distribution is used. By using this kind of a device, the white standard surface which has a known spectral distribution and low optical absorption and the specimen of which the color is measured are measured subsequently. The ratio **R(λ)** of the reflected light value measured from the specimen which is measured in the expected narrow wavelength to the reflected light value measured from the white standard surface is obtained. After obtaining these, coefficients, the tristimulus color coordinates are measured as shown in the mathematical calculation below:

$$X_{10} = \frac{100 \int_{380}^{780} S(\lambda)\overline{x}_{10}(\lambda)R(\lambda)d\lambda}{\int_{380}^{780} S(\lambda)\overline{y}_{10}(\lambda)R(\lambda)d\lambda}$$

[1a]

$$Y_{10} = \frac{100 \int_{380}^{780} S(\lambda)\overline{y}_{10}(\lambda)R(\lambda)d\lambda}{\int_{380}^{780} S(\lambda)\overline{y}_{10}(\lambda)R(\lambda)d\lambda}$$

[1b]

$$Z_{10} = \frac{100 \int_{380}^{780} S(\lambda)\overline{z}_{10}(\lambda)R(\lambda)d\lambda}{\int_{380}^{780} S(\lambda)\overline{y}_{10}(\lambda)R(\lambda)d\lambda}$$

[1c]

In these CIE 1964 formulas for standard observer with **10˚** angle of observer: **S(λ)** - Relative spectral power distribution of the illuminant, $\overline{x}_{10}(\lambda)$, $\overline{y}_{10}(\lambda)$, $\overline{z}_{10}(\lambda)$. Color matching functions for CIE **10˚** Supplementary Standard Observer. These values are determined by CIE in ranges of 1 nm and are available in tables.

After the calculation of the Formulas [1a-1c], **xyz** color coordinates are computed as shown below:

$$x_{10} = \frac{X_{10}}{X_{10} + Y_{10} + Z_{10}} \qquad \text{[2a]}$$

$$y_{10} = \frac{Y_{10}}{X_{10} + Y_{10} + Z_{10}} \qquad \text{[2b]}$$

$$z_{10} = \frac{Z_{10}}{X_{10} + Y_{10} + Z_{10}} = 1 - x_{10} - y_{10} \qquad \text{[2c]}$$

When necessary, the color differences between the two specimens can be calculated by analytic operations as given in Formulas [2a-2c].

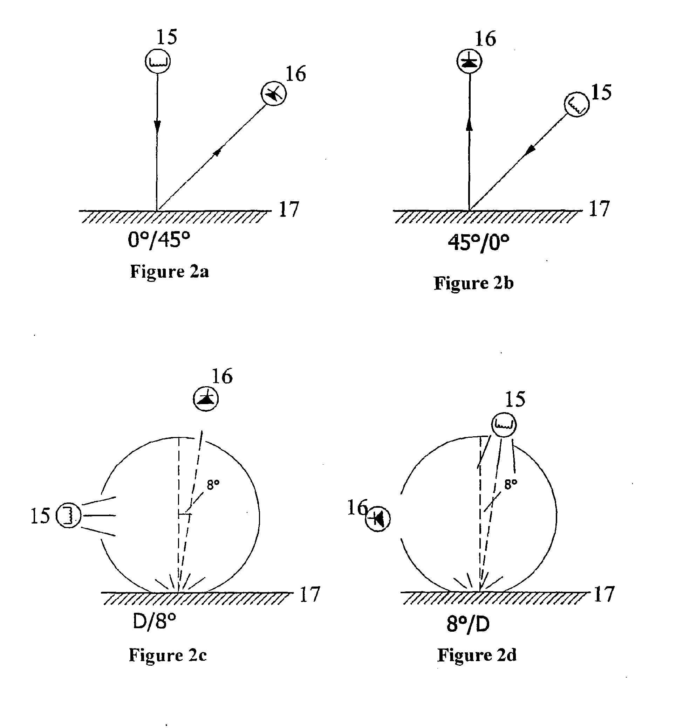

Lighting and observation geometry standards are specified for calculating the reflection coefficient of the specimens in color measurements. In these measurements, either **0°/45°** or **45°/0°** measurement geometry is used (Figure 2a and 2b). As seen in figure 2a, the light source lights the specimen with a **0°** angle and the light detector detects some of the light reflected from the specimen with **45°** angle. In **45°/0°** geometry, (Figure 2b) the light source lights the specimen with a **45°** angle and the light detector detects the light reflected from the specimen observing it from **0°** angle.

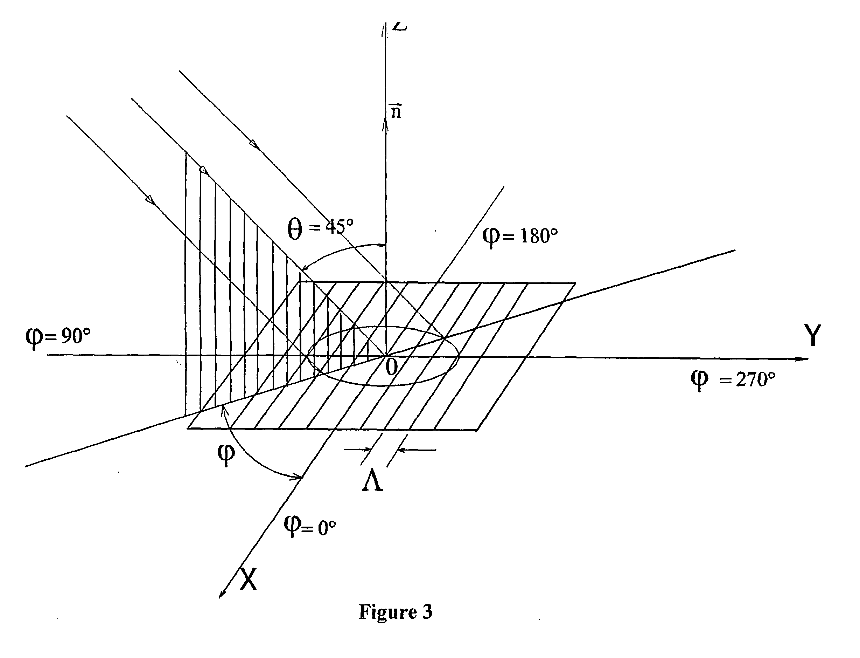

Provided that other parameters are the same, the devices which use both measurement geometries measure the color with high sensitivity and correctness in Lambert type materials which reflect the projected light. The reason behind can be understood by looking at the reflection process at modified three dimensional polar coordinate system of for example **45°/0°** geometry (Figure 3). Here, the illumination angle $\theta = \mathbf{45°}$ is as shown in Figure 2b. In optical processes, the angles are measured from the normal vector n of the surface perpendicular to the illumination point. The angle between the intersection of the illumination on the specimen surface and any specified direction on the specimen is the Azimuth $\varphi$ angle. When the specimen is smooth and Lambert type, the reflection coefficient used in Formula [1] $R(\lambda)$, is same in all $\theta$ and $\varphi$ angles. The distribution of the indicatrix of reflection shown in Figure 3, form a semi sphere with the center in O point and in the direction of **n** vector. This is an assumption derived from the definition of Lambert type materials. In these types of materials, if the color measurements are made at any $\varphi$ angle, the reflection coefficient will be the same and no change will be observed in color measurements.

Materials with Lambert type reflections are ideal materials. But in materials we use in daily life, this specialty is not dominant. Generally, the distribution of the indicatrix of reflection of materials $R(\lambda, \theta, \varphi)$ has three variables. Along with the wavelength, the illumination angle $\theta$ and the angle $\varphi$ with respect to the materials position are the other variables. Reflection coefficients of any type of material in the region of 380 nm and 780 nm to upper semi sphere can be found by triple integration. For example, total energy reflected to all directions from an ideally white, standard, optically nonabsorbent surface is equal to the $E_{in}$ energy of the source illuminating this surface with a beam of light in the range of 380 nm and 780 nm.

$$\int_{380}^{780} \int_{0}^{90} \int_{0}^{360} R_{WS}(\lambda, \theta, \varphi) d\lambda d\theta d\varphi = E_{in}$$

In measurement geometries shown in figures 2a and 2b, the angle at $\theta = \mathbf{45°}$ (**0°/45°**) and $\theta = 0°$ (**45°/0°**) is kept constant. In rough materials where the angular distribution of reflection quotient is disordered, the color measurements are effected by the materials position in the device ($\varphi$ angle), and when this position changes from $\varphi_1$ to $\varphi_2$, the reflection

quotient at these angles become $R(\lambda,\theta,\varphi_1) \neq R(\lambda,\theta,\varphi_2)$, thus, the measurement results can not be repeated.

Therefore, with rough and non-monochrome materials **D/8˚** or **8˚/D** measurement geometry is used. An integration sphere has to be used in order to realize this geometry. Integration spheres are devices which have an entrance hole (for the light source), an exit hole (for the receptor), a hole for specimen replacement; the inner surfaces covered with a Lambert type white material and are generally made of metal. **D/8˚** geometry is shown in Figure 2c. The inner surface of the sphere is diffused illuminated (**0˚ ≤ θ ≤ 90˚**) by diffused reflection by the light coming from the illumination source and the light which is reflected at **8˚** angle which is a portion of the light reflected from the specimen exits through the exit hole and the spectral density distribution is measured by the receptor. **8˚/D** geometry is shown in Figure 2d. Here, the illumination source illuminates the specimen with an θ = **8˚** angle. The light reflected from the specimen is diffuse-reflected from the sphere walls and is measured at the exit hole by the receptor.

The advantage of using color measurement devices with integration spheres (especially working with **D/8⁰** geometry) in rough materials is to average the disorders arising from the reflection coefficient $R(\lambda,\theta,\varphi)$ by diffused reflections. In other words, bringing the three variable reflection $R(\lambda,\theta,\varphi)$ function to one wavelength $R(\lambda)$ as hardware by diffuse reflections.

Some disadvantages of using integration spheres are listed below. Firstly, compared to angular geometries it is known that integration spheres are sensitive to higher color changes (low sensitivity).The illumination source used in these systems have to be very powerful and the proportion of the spheres diameter has to be as large as possible to the specimen hole. Even when there is no specimen (reflection), some of the light is reflected and measured by the receptor (Figure 2c), or the light directed from the illumination source to the specimen hole being reflected like a background light from the specimen hole (Figure 2d) makes the measurements complicated especially with dark colored specimens.

In order to repeat the color measurements in rough materials, R.Hunter advices to make another calculation by rotating the specimen's position **90˚** and then taking the arithmetic mean of the two calculations, in his book "The Measurement of Appearance" (John Wiley & Sons, 1975, p.257). In the education web site of DATACOLOR INC. company, it is advised to turn the specimen **90˚** four times and take the mean. The analytical figure of this tristimulus $\overline{x}_{10}\,(\lambda)$ value calculation is given below:

$$\overline{x}_{10} = \frac{100}{4\int\limits_{380}^{780} S(\lambda)\overline{y}(\lambda)\,d\lambda}\left[\int\limits_{380}^{780} S(\lambda)\overline{x}(\lambda)R(\lambda,\theta,\varphi)\Big|_{\substack{\theta=Const\\\varphi=\varphi_i}}\,d\lambda + \int\limits_{380}^{780} S(\lambda)\overline{x}(\lambda)R(\lambda,\theta,\varphi)\Big|_{\substack{\theta=Const\\\varphi=\varphi_{i+90}}}\,d\lambda \right.$$

$$\left. + \int\limits_{380}^{780} S(\lambda)\overline{x}(\lambda)R(\lambda,\theta,\varphi)\Big|_{\substack{\theta=Const\\\varphi=\varphi_{i+180}}}\,d\lambda + \int\limits_{380}^{780} S(\lambda)\overline{x}(\lambda)R(\lambda,\theta,\varphi)\Big|_{\substack{\theta=Const\\\varphi=\varphi_{i+270}}}\,d\lambda \right]$$

[3]

To obtain the Tristimulus $\overline{y}_{10}\,(\lambda)$ value, replacing standard value $\overline{y}(\lambda)$ by $\overline{x}(\lambda)$ in formula[3] and for Tristimulus $\overline{z}_{10}\,(\lambda)$ value replacing standard value $\overline{z}(\lambda)$ by $\overline{x}(\lambda)$ in the same formula is enough.

Here, $\varphi=\varphi_i$ is the azimuth angle of the specimen at any first position "i". With this method, only materials (for example jeans, thread wound regularly on reel etc.) with roughness disorder with surface period $\Lambda$ (Figure 3) can be repeatedly measured. This process is not advised in terms of time consumption (the device is manually operated and the final measurement takes four times more time because of calculating the mean) and accuracy (turning the specimen **90˚** degrees and replacing the same areas in front of the color measurement unit). With this method, repeated color measurements can not be made concerning materials with roughness disorder (for example velvet, cotton, leather, thread wound regularly on reel etc.)

Another method is to illuminate the specimen homogeneously from different directions. This method is used in the D25M and D25L devices of HUNTERLAB Company. In these devices, a light from a source illuminates the specimen with a circular light at an **45˚** angle (θ=45˚ , **0˚ ≤ φ ≤ 360˚** ). A similar illumination is defined in an US patent US5822053 where light emitted diodes (LED) which are on a fixed base and spread light in a set sequence and a plate which will diffuse light and is placed in front of them is proposed. Usage of such illumination systems is inappropriate to the CIE color measurement rules. Taking into consideration the insufficient light value proposed and the control difficulty of the homogeny of the illuminated surface, it would not be advised to apply the method proposed in this patent.

In the European Patent No.EP1072884, the LEDs are placed circular on a plate and a circular Fresnel lens is used in front of them. The aim is to illuminate a specific area equally from different direction. Here, the Fresnel lens could only help for decreasing the systems scale. Because the illumination bundle is at **45+/-2˚** angle, usage of such illumination is inappropriate to the CIE color measurement rules.

US patent US4881811 is advised for the color measurements of monochrome and rough surfaces such as human tooth or skin. The color measuring unit used here contains two integration spheres placed one after the other and connected by two channels. The light reflected from the specimen reflects from the spheres and reaches the spectrophotometer entrance and the spectrum is measured here. The reference spectrum measure is taken from the rotating supplementary mirror in the second sphere. The weak points of the device is common in all devices using the integration sphere; in order to achieve high sensitivity, the diameter of the sphere has to be as large as possible. This necessity affects the dimension and weight of the device. White spheres used in industrial environments get dirty quickly and due to cleaning, the special inner cover is damaged. If the specimen has a dark color, the intensity of the background light reaches the intensity of the light reflected from the specimen and the correctness of the measurement decreases.

European Patent No.EP0570003 discloses a color measuring device for colored material in the form of texture, thread or particle. Said device comprises a color measurement unit which illuminates the measurable field of view at a geometry of 45˚/0˚; a computer or built-in digital processor with software; an electrical motor with angular encoder and electronics circuit; a table which is connected to said motor, is designed to be fixed or continuously rotated with a constant and predetermined velocity. But in this invention the color of a moving or large sample of textured materials can not be measured.

Japan Patent No. JP4140620 a sample illuminating optical system is provided on an extension line of a rotary shaft above a sample. An optical axis of the optical system is made coincident with the center line of the rotary shaft. An optical axis of a photo detecting optical system intersects the center line of the rotary shaft at the surface of the sample, with assuming **45˚** from the center line. Primaries filters are sequentially exchanged, so that the components of primaries of the reflecting light are extracted and brought into a photo detecting element. A control device controls a sample stage driving device to rotate a sample stage every **45˚** in one direction. The control device controls also a filter exchanging mechanism at each angular position to sequentially exchange the filters. An output of the element when the light passes each filter is stored in a memory by the control device. After the sample is rotated once, outputs of the primaries from the photo detecting element stored in the memory are added and averaged, which becomes the measured value of the reflecting light of the primaries of the sample. This invention proposes to use measurements from every **45˚** of the azimuth angle of a specimen.

As a result of the above mentioned causes, the color measurements can not be made repeatability in materials which are either monochrome or colorful and have a rough surface (for example, velvet, cotton, jeans, ornamented material leather, plastic, any material in the form of particles) and consequently, the color appropriateness processes are all done with the help of controllers which are sensitive to color differences.


### Aim of the Invention

The aim of this invention is to measure the color and color differences in materials with high sensitivity and repeatability, within the boundaries of CIE regulations and without using human eye.

The color measuring device designed for realizing the above mentioned aim in accordance with the invention is defined in claim 1.


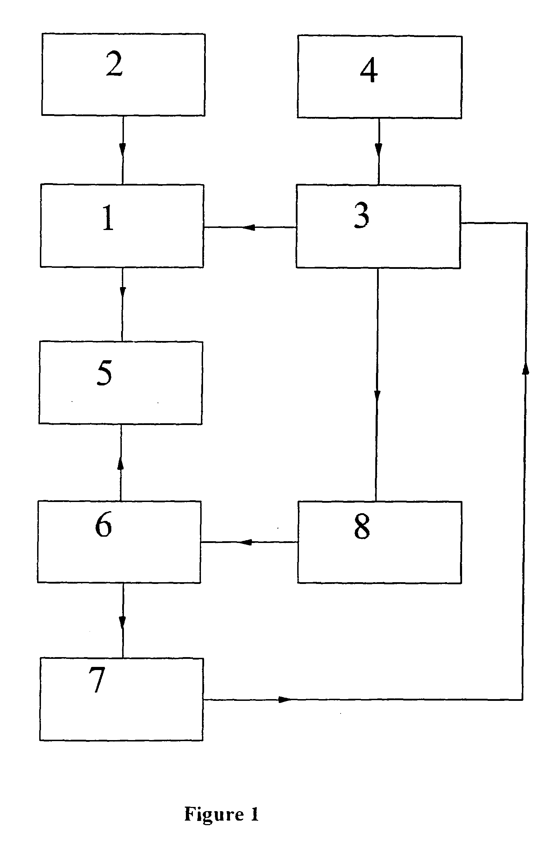

Figure 1 - The block diagram of the color measuring device.

Figure 2 a, 2b, 2c, 2d - The illumination and observation geometries of the specimen used in color measuring

Figure 3 - The view of the **45˚ / 0˚** color measuring geometry in polar coordinates

Figure 4a - The perspective view of the rotating table

Figure 4b - The perspective view of the specimen holder which has a cut circular shape

Figure 4c - The side section view of the rotating table with the specimen mounted

Figure 5a - The top view of the thread wound regularly on reel

Figure 5b - The side section view of the rotating table with reel

Figure 6a - The measurement-time diagram of the device using photodiode receiver

Figure 6b - The measurement-time diagram of the device using CCD receiver

Figure 7 - The graphic depiction of the device measuring color and color differences with moving mirrors


The color and color differences measurement device comprises, a supply unit (2); a unit (1) which runs by any method of spectrophotometry and tristimulus, which measures spectral intensity distribution using **0˚ / 45˚ or 45˚ / 0˚** geometries (Figure 2a and 2b) and which continuously (CW) and temporarily measures color by illumination; a computer or built-in digital processor (3) which converts the reflected spectral coefficients and illumination conditions it obtained for a given time from color measurement unit to color coordinates by mathematical calculations and which ensures regular operation of the whole device; a software (4) running under this processor (3) which converts colors and color differences to digital knowledge in accordance with standards and which ensures the complex operation of the device;

a rotating table (5) with a preferably circular tray (9) whose angular position is determined by an angular encoder (7), and which is run by an electrical motor (6) whose rotation is adjustable through electronic circuits (8); and a specimen holder (10, 13) which ensures the positioning of specimens on this table during measurement.

Theoretical background of measuring the colors and color differences of the specimens placed on the rotating table (5) are explained below in detail together with related mathematical computations.

Generally light sensors used in color measurement units operate with a specific velocity and sensitivity. Owing to this, integration time of the detector signal forms in a definite time period $T°_{in}$. Operating speed and sensitivity factors of light sensors which are based on different optoelectronical principles also variation.

Photodiodes or photodiode array type of devices convert the incident radiation to photocurrent (which in turn can be converted into photovoltage by passing it over a resistance) that passes over the circuit controlling this device with a very low time constant. Any sufficiently sensitive amperemeter (voltmeter) reading the outputs of this device, sends this raw information to processing circuits with the frequency of reading. Consequently, a continuously flowing photocurrent is obtained against the continuous incident light on this device.

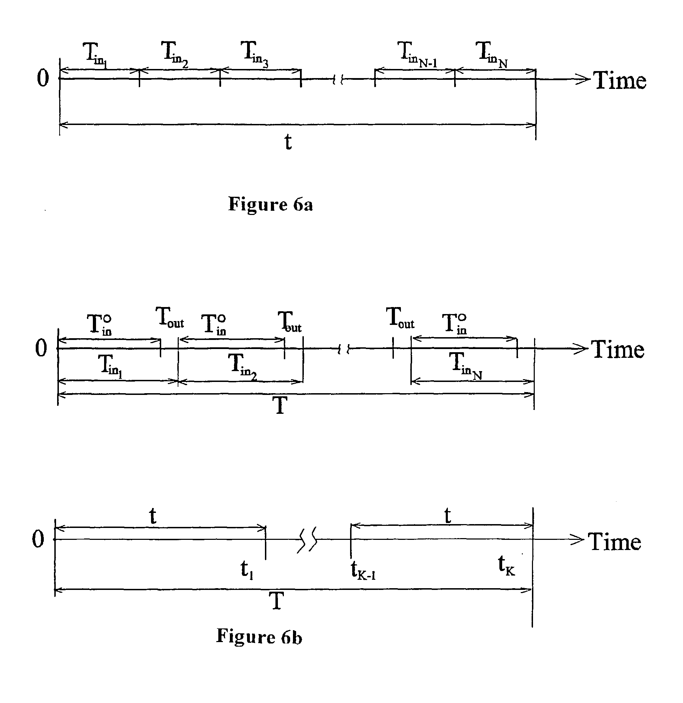

If reflection intensity spectra of specimens are measured as $T_{specimen}(\lambda,\theta,\varphi)$ with the invented device and white standard plate reflection intensity spectra are $T_{white}(\lambda,\theta,\varphi)$, and measuring time with continuously rotating photo-diode type detectors is $T_{in}$ and the time it took to complete an exact turn is $t$, then it is possible to measure exact $N$ when rotating table (5) turns **360°**, by adjusting the rotation speed of motor (6) with processor (3) (Figure 6a). After these measurements are converted into numbers and arithmetically averaged, it is possible to convert $R(\lambda,\theta,\varphi)$ function with three variables into a function of $R(\lambda)$ with only one wavelength variable, through numeric operations:

$$R_{PD}(\lambda) = \overline{R}_{PD}(\lambda,\theta,\varphi) = \frac{\sum_{i=1}^{N} T_{specimen}(\lambda,\theta,\varphi_i)\Big|_{\substack{\theta=Const\\\varphi_i}}}{\sum_{i=1}^{N} T_{white}(\lambda,\theta,\varphi_i)\Big|_{\substack{\theta=Const\\\varphi_i}}} \qquad [4]$$

After the operations stated in Formula [4] are done, Formulas [1, 2] which are a standard procedure, can be applied.

If Linear or 2D Charge Coupled Devices (CCD) or Charge Injection Devices (CID) are used in color measuring unit, measurement- time diagram is different. Incident light on CCD or CID type devices is converted into electric charge within the pixels forming these devices. Level of electric charge formed in unit time in any one of pixels is directly proportional with the incident light intensity (photon number) and this is determined by the light-charge conversion performance of the device. Before the charge level formed on devices operating under conditions of continuous illumination reaches to saturation, pixels are passed into reading mode from light detection mode for the proportionate operation of the device, and raw information detected by the line of pixels is read one by one. Under certain illumination conditions, if integration time of CCD detecting signal is formed within $T°_{in}$ time period and reading time of this line is $T_{out}$, one period integration time for this sensor becomes $T_{in} = T°_{in} + Tout.$

Generally it is possible to take $T_{out} \ll T°_{in}$ in contemporary electronic devices. Under these conditions, operation of the device assumes that continuously incident light is in integration mode.

Since the reflected spectra of the specimens are in a continuously rotating mode with the device subject to this invention, specimen turns into the angle $\varphi_{in}$ in time $T_{in}$ and meanwhile irregularities of the all angular reflection coefficients from $\varphi_{in}$ azimuth angles of the specimen are reflected on the measured signal. Analytical description of the signals collected on pixels of the device means integration. Rotation angle of the specimen and the time it took for this is directly proportional with the angular velocity $\omega$ of the motor (6) turning the rotating table (5):

$$\varphi_{in} = \omega\, T_{in} \qquad [5]$$

When signal to noise ratio (SNR) formed during measurement is insufficient, related measurements are repeated (i.e. N times). According to Nyquist theorem, compared to one time measurement, the average of N times measurements increases as of signal to noise ratio: **SNR ~ √N**. Time for the formation of this signal is **T** and the rotation angle of the specimen is $\varphi$ (Figure 6) :

$$T = N\ T_{in} \qquad\qquad [6]$$

$$\varphi = \omega\,T = \omega\ N\ T_{in} = N\ \varphi_{in} \qquad\qquad [7]$$

If complete turn of the rotating table (5) takes the time **t**, then the time needed for the system for an average measurement:

$$T = K\,t = N\ T_{in}\ ; \qquad\qquad [8]$$

where **K** and **N** may be any integer. As can be seen from the Formula [8], one measurement time **T** is equal to **t** when rotating table (5) turns a complete turn and **K = 1**. Formed coefficients in Formula [8] are reflected to the software (4) of the device and the software (4) provides a higher performance of repetitive measurements for the device by adjusting the angular velocity $\omega$ of the rotating table motor (6).

The device subject to this invention, and which measures colors and color differences is literally in accordance with the color measuring procedures of International Comission of Illumination for spectral intensity distribution measurements. Different from the other devices that measure reflection coefficients **R($\lambda$)** from a specimen placed in any azimuth angle without using them in tristimulus values ([1]) or that make measurements with a total of 4 different azimuth angles by rotating **90˚** and then averaging the values, the device subject to this invention detects and averages reflected continuous signals from all existing azimuth angles of the specimen. When **K=1**, averaging procedure becomes integration with angular variable:

$$R_{CCD1}(\lambda) = \overline{R}_{CCD1}(\lambda,\theta,\varphi) = \frac{\displaystyle\int_0^{360} T_{specimen}(\lambda,\theta,\varphi)\Big|_{\theta=Const}\,d\varphi}{\displaystyle\int_0^{360} T_{white}(\lambda,\theta,\varphi)\Big|_{\theta=Const}\,d\varphi} \qquad [9]$$

If a higher SNR value is required to be obtained among the measured values, measurements should be carried out within the time period of several turns of rotating table. In this case, Formula [5] is used and angular integration in Formula [8] can be converted into integration with a time variable:

$$R_{CCD}(\lambda) = \overline{R}_{CCD}(\lambda,\theta,\varphi) = \frac{\displaystyle\int_0^{T} T_{specimen}(\lambda,\theta,\omega t)\Big|_{\theta=Const}\,dt}{\displaystyle\int_0^{T} T_{white}(\lambda,\theta,\omega t)\Big|_{\theta=Const}\,dt} \qquad [10]$$

Method of measuring colors and color differences of this invention subject is to convert reflection coefficients of **R($\lambda,\theta,\varphi$)** with three variables into reflection coefficient of **R($\lambda$)** with one variable by using the invented device measuring colors and color differences. Color coordinates computed with this method are measured repeatability in rough materials.

The method of measuring color and color differences is applied in varying ways depending on the type of photodetector used in the invented device measuring colors and color differences.

In the case of using photodiode type photodetector in color measurement unit, calibrated white standard plate of the device is fixed on the continuously rotating circular tray (5) and **T($\lambda$)$_{white}$** spectral distribution of the reflected light intensity is measured by the illuminating color measurement unit (1). In any narrow spectral band of the related measurement, care is taken for it to be lower than the saturation level. This procedure is done for only one time by adjusting the intensity of light with electric current or optical filter. If reflection spectra of specimens are measured with continuously rotating photodiode type detectors as $T_{in}$ and the time it took to complete an exact turn is $t$, then exact $N$ is measured when rotating table (5) turns 360˚, by adjusting the rotation speed of motor (6) or reading frequency with processor (3).

With the same method, specimen is fixed onto the circular tray (5) rotating continuously and then the spectral distribution of the reflected light intensity $T(\lambda)_{specimen}$ is measured $N$ times by illuminating color measurement unit through rotating the specimen with a certain velocity. These measurements are digitized and then calculating through Formula [4], $R(\lambda,\theta,\varphi)$ function with three variables is converted into $R(\lambda)$ function with one variable. Since $SNR$ is ~ $\sqrt{N}$, depending on the roughness of the material, number of $R(\lambda)$ measurements $N$ performed during an exact turn should be sufficiently large. In order to achieve the desired $SNR$ in measurements, they are done during several exact turns.

The standard procedure of Formula [1-2] is applied after Formula [4] operations are performed.

In the case of using CCD type photo detector in the color measurement unit, calibrated white standard plate of the device is fixed onto the continuously rotating rotating table (5) and $T(\lambda)_{white}$ spectral distribution of the reflected light intensity is measured by the illuminating color measurement unit. Care is taken for any narrow spectral band of the related measurement to be lower than the saturation point. This operation is performed by adjusting signal integration time $T_{in}$ of the CCD type detector. Meanwhile, rotation angle information of the rotating table (5) at the related measurement time is sent by encoder (7) to the computer or built-in processor. According to this information, computer (3) adjusts the speed of motor (6) via electronic circuit (8). After adjusting rotation velocity of specimen in the first step, during the time period for the rotating table (5) to turn an exact **360˚** rotation **(K=1) N** measurements are performed.

Using the same method, specimen is fixed onto the continuously revolving circular rotating table (5) and $T(\lambda)_{specimen}$, spectral distribution of the reflected light intensity is measured by illuminating color measurement unit (1) $N$ times. These measurements are digitized and then $R(\lambda,\theta,\varphi)$ function with three variables is converted into $R(\lambda)$ with only one wavelength variable through numeric operations by use of Formula [9].

If a higher $SNR$ value is required to be achieved among measured values, measurements are performed for the time period of two exact turns (**K=2**) of the rotating table. In this case, $R(\lambda)$ function with only one wavelength variable is calculated by Formula [10].

In order to achieve the desired $SNR$ values in measurements, they should be performed for t time of several **K** exact turns. In this case, $R(\lambda)$ function with only one wavelength variable is calculated by Formula [10].

The standard procedure of Formula [1-2] is applied after Formula [4] operations are performed.

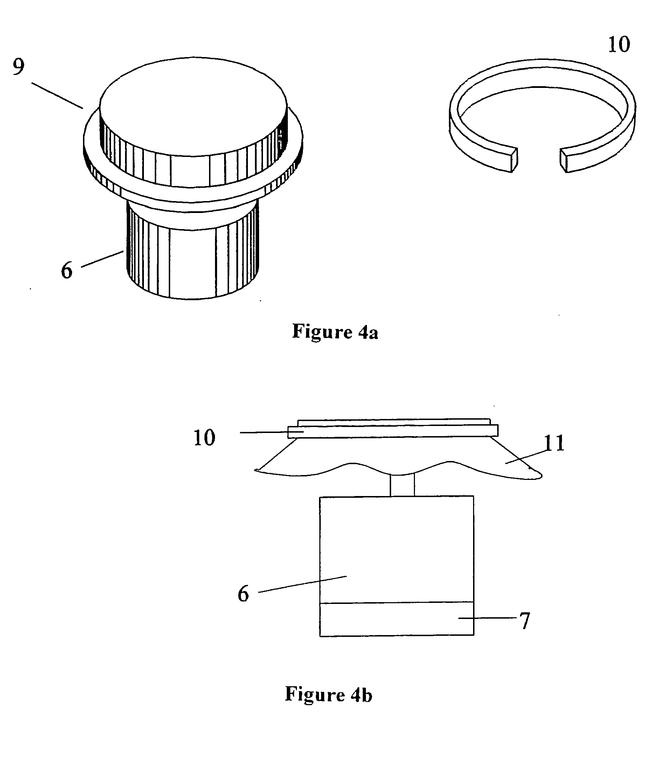

Another device which measures colors and color differences contains different specimen holders (10, 13) varying according to the type of specimen to be measured. Specimens are fixed onto rotating table (5) flat and without wrinkles (Figure 4c) with the specimen holder (10) in the form of a truncated ring used for texture specimens (11) (Figure 4b). Specimen holder in the form of a truncated ring (10) is made sufficiently flexible in a way not to be affected by the structure and thickness of the specimens.

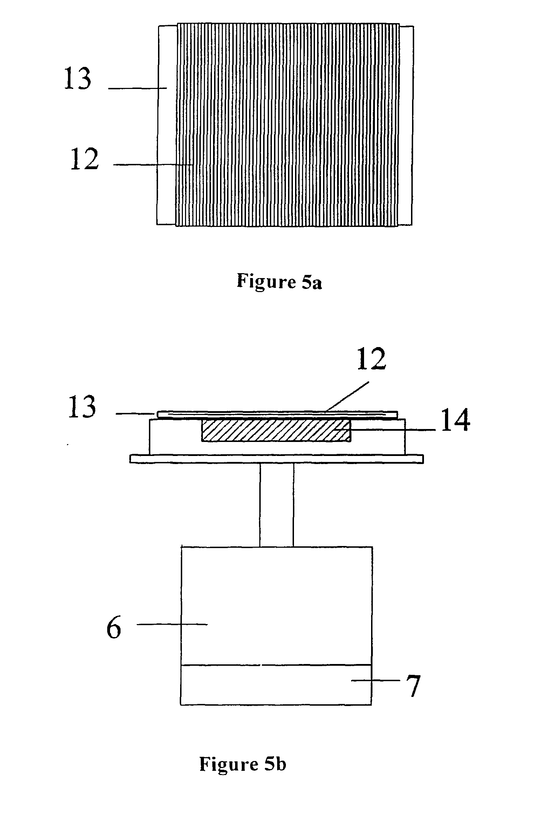

The reel (13) used as specimen holder (10, 13) for thread specimens (12), is at first made from thin, galvanized iron sheet preferably in a square form and then painted in dull black to restrain reflection in he lowest level. Thread specimens (12) are wound on this reel (13) evenly and adjacently (Figure 5a). Afterwards it is placed on the permanent magnet (14) carrying tray (9) of the circular rotating table (5) (Figure 4a). The reel (13) being made of thin iron sheet is an important aspect of the accurateness and repeatability of the measurements.

Materials in particle forms (granule, powder, rice, flock etc.) are placed on the continuously rotating circular tray after they are compressed into a transparent container with a standard force.

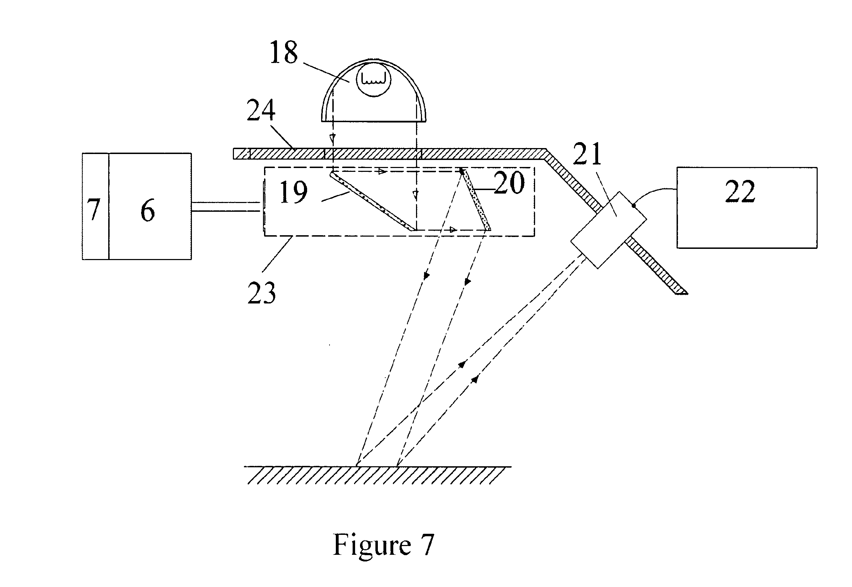

When the specimens could not be placed on a moving rotating table or on a specimen holder due to any reason, or when color measurement has to be made during a production process, and can be placed above a stationary specimen, device can be mounted in a different way (Figure 7).

In such case, the device according to the invention comprises :

- a light source (18) above a plate (24), illuminating a first mirror (19) with a predetermined angle;
- said first mirror (19) mounted on a rotatable support (23), beneath said plate (24), and being placed in an inclined position to direct the light onto a second mirror (20);
- said second mirror (20) mounted on said rotatable support (23), beneath said plate (24), and being placed in an inclined position to direct the light onto said specimen;
- lenses (21) connected to the plate (24) in such a way that the reflected light from an illuminated specimen with an angle of 45˚ is directed to color measurements unit (22) measuring reflected spectrum in a 0˚/45˚ geometry.
- an electric motor (6); whose rotor angular velocity being adjusted by electronic circuits and whose rotor angular position being determined by an angular encoder (7); enabling to rotate the double mirrors with adjustable rotational velocity and in order to illuminate a specimen, continuously, from different consecutive azimuth positions during rotation.

Said device can be used; for measuring color coordinates and color differences of all kinds of monochrome or colorful textured material either in thread or particle form, or monochrome and polychrome rough specimens, which are illuminated by an illumination light under an angle of 0° +/- 10°, by acquiring their reflectance spectrums from the desired area to be measured.

Depending on the detector type used in the color measurement unit (22), in these measurements R($\lambda$) reflection coefficients are calculated with the methods determined by formula [4] and formula [9,10] which are used with photodiode or photodiode arrays, working in the photovoltaic or photoconductive mode of operation, and CCD's or CID's or any kind of charge integrating photosensors. Afterwards, color coordinates are determined through calculations in formula [1] and [2] in accordance with the standards.

In order to perform the required color measurement, device preferably, integrated with a computer or built-in digital processor with software.

According to the predetermined acquisition and digitizing time of the color measurement unit (22), a certain integer number of measurements are performed during the period of an exact 360° turn or a few full turns of the rotatable support (23).

Said device also enables its color measurement unit (22) and digitizing time being possible to measure and adjust while the rotatable support (23) is continuously rotated at a constant and certain predetermined velocity, so that the reflectance spectrum can be measured at every azimuth angle.

By means of said predetermined velocity, color measurement unit's (22) acquisition and digitizing time are set to specific values which provide a certain integer number of measurements and the quantification of the reflection spectrum at every azimuth angle during an exact 360° turn or a few full turns while the measured reflectance is normalized by means of reflection spectrum of the white standard plate so that the three variable reflection coefficients are converted into a single variable reflection coefficients as a result.

Alternatively the velocity is obtained in accordance with the predetermined acquisition and digitizing time of the color measurement unit (22), providing a certain integer number of measurements and the quantification of the reflection spectrum at every azimuth angle during an exact 360° turn or a few full turns, while the measured reflectance is normalized by means of reflection spectrum of the white standard plate so that the three variable reflection coefficients are converted into a single variable reflection coefficients as a result.

The above selected applications are explained for a better understanding of the invention and they are not in qualities that restrict the protection scope of the invention. Along with the information explained about the invention, modifications to be performed on these preferred applications should be considered within the protection scope of the invention.

**Claims**

1. A color measuring device; for measuring color coordinates and color differences of all kinds of monochrome or colorful textured material either in thread or particle form, or monochrome and polychrome rough specimens, which are illuminated by an illumination light under an angle of 0° +/- 10°, by acquiring their reflectance spectrums from the desired area to be measured;

   **characterized in that**,
   it can be used when a specimen could not be placed on a moving rotating table or on a specimen holder due to any reason or when color measurement has to be made during a production process and can be placed above a stationary specimen
   and comprises:

   - a light source (18) above a plate (24), illuminating a first mirror (19) with a predetermined angle;
   - said first mirror (19) mounted on a rotatable support (23), beneath said plate (24), and being placed in an inclined position to direct the light onto a second mirror (20);
   - said second mirror (20) mounted on said rotatable support (23), beneath said plate (24), and being placed in an inclined position to direct the light onto said specimen;
   - lenses (21) connected to the plate (24) in such a way that the reflected light from an illuminated specimen with an angle of 45° is directed to color measurements unit (22) measuring reflected spectrum in a 0° / 45° geometry,
   - an electric motor (6); whose rotor angular velocity being adjusted by electronic circuits and whose rotor angular position being determined by an angular encoder (7); enabling to rotate the double mirrors with adjustable rotational velocity and in order to illuminate specimen, continuously, from different consecutive azimuth positions during rotation.

**2.** A device according to claim 1, wherein the color measurements unit (22) is CCD.

**3.** A device according to claim 1, wherein the color measurements unit (22) is CID.

**4.** A device according to claim 1, wherein the color measurements unit (22) is any kind of charge integrating photosensors.

**5.** A device according to claim 1, wherein the color measurements unit (22) is photodiode or photodiode arrays, working in the photovoltaic or photoconductive mode of operation.

**6.** A device according to claim 1. in order to perform the required color measurement, device preferably, integrated with a computer or built-in digital processor with software.

**7.** A device according to claim 1, wherein, by means of predetermined velocity, color measurement unit's (22) acquisition and digitizing time are set to specific values which provide a certain integer number of measurements and the quantification of the reflection spectrum at every azimuth angle during an exact 360˚ turn or a few full turns while the measured reflectance is normalized by means of reflection spectrum of the white standard plate so that the three variable reflection coefficients are converted into a single variable reflection coefficients as a result.

**8.** A device according to claim 1, wherein, the velocity is obtained in accordance with the predetermined acquisition and digitizing time of the color measurement unit (22), providing a certain integer number of measurements and the quantification of the reflection spectrum at every azimuth angle during an exact 360˚ turn or a few full turns, while the measured reflectance is normalized by means of reflection spectrum of the white standard plate so that the three variable reflection coefficients are converted into a single variable reflection coefficients as a result.

**Patentansprüche**

**1.** Eine Vorrichtung zum Messen der Farben, die bei der Messung vom befärbten oder mit einer Farbfläche ausgerüsteten, harten Material, welches als Faden oder Partikel vorkommt, verwendet wird, sowie bei der Messung von Koordinaten oder Farbunterschieden der einfarbigen und bunten Mustern, die unter einer Winkel von 0˚ +/- 10˚ durch das Beleuchtungslicht bestrahlt werden, indem man aus dem zu bemessenden Bereich Widerscheinspektren hervorgehen lässt, ist **dadurch gekennzeichnet;**
**dass** die erwähnte Vorrichtung angewandt oder auf die ein unbewegliches Muster gelegt oder während der Messprozesses der Farben im erforderlichen Fall die benannte Vorrichtung betrieben wird, wenn es unmöglich ist, aus irgend einem Grund, ein Muster auf die beweglich drehbare Platte hinzusetzen oder dieses einem Musterbehälter anzuschließen; und enthält:

- eine Lichtquelle (18), welche mit einer vorangegebenen Winkel einen ersten Spiegel (19) bestrahlt und auf einer Platte (24) befestigt ist;
- den schon benannten ersten Spiegel (19), der zur Umlenkung des Strahles auf den zweiten Spiegel (20) in einer schrägen Position zugeordnet wurde, welcher auf einer Stütze (23) liegt, die unter die oben erwähnte Platte (24) drehbar angeordnet ist, und
- den schon benannten zweiten Spiegel (20), der zur Umlenkung des Strahles auf das schon beschriebene Muster in einer schrägen Position zugeordnet wurde, welcher auf einer Stütze (23) liegt, die unter die oben erwähnte Platte (24) drehbar angeordnet ist.
- Linse (21), die mit der Platte (24) so verbunden sind, dass sie das aus dem bestrahlten Muster mit einer Winkel von 45˚ widerspiegelte Licht in die Farbmessungseinheit (22) umlenkt, wobei die Messung des Widerscheinspektrums in einem geometrischen Messbereich zwischen 0˚ /45˚ resultiert;
- Einen elektrischen Motor (6), der den Vorgang ermöglicht, dass die Drehgeschwindigkeit des Spiegelpaars verstellbar und die beispielsweise aus aufeinander folgenden Azimutlagen hervorkommende Beleuchtung kontinuierlich ist, wobei die Geschwindigkeit der Winkelumlenkung durch elektronische Schaltungen verstellt wird und die Lage der Winkelumlenkung durch ein Winkelkodierungsmittel (7) festgestellt wird.

**2.** Eine Vorrichtung nach Anspruch 1, wobei die Farbmessungseinheit (22) CCD ist.

**3.** Eine Vorrichtung nach Anspruch 1, wobei die Farbmessungseinheit (22) CID ist.

**4.** Eine Vorrichtung nach Anspruch 1, wobei die Farbmessungseinheit (22) aus irgendwelchen Fotoempfängern besteht, die wohl Leistungszusammenführer sind.

**5.** Eine Vorrichtung nach Anspruch 1, wobei die Farbmessungseinheit (22) über ein photovoltaisches oder photoleitfähiges Betriebsystem bewerkstelligt wird, welches aus Photodioden besteht oder eine Photodiodenreihe bildet.

**6.** Eine Vorrichtung nach Anspruch 1, wobei zur Bewerkstelligung der erwünschten Farbmessung die Vorrichtung vorzugsweise mit einem digitalen Datenverarbeiter verbunden ist, der anhand eines Computers oder einer Software eingeschaltet wird.

**7.** Eine Vorrichtung nach Anspruch 1, wobei durch die vorangegebene Geschwindigkeit die Zieten der Aufnahme und des zählerischen Bewerten von der Farbmessungseinheit (22) ein bestimmtes Plenum und eine Umdrehung des Widerscheinspektrums um 360˚ verstellt wird oder nach einigen vollendeten Umdrehungen sie den bestimmten Werten angepasst werden, welche bei jeder Azimutwinkel den Messvorgang ermöglicht, andererseits der bemessene Widerschein mit dem Spektrum des weißen standardisierten Platten so normalisiert wird, dass diese drei veränderlichen Koeffizienten des Widerscheins schließlich nur in ein veränderliches Koeffizient des Widerschein umgewandelt werden.

**8.** Eine Vorrichtung nach Anspruch 1, wobei die Zieten der Aufnahme und des zählerischen Bewerten von Farbmessungseinheit (22) ein bestimmtes Plenum und eine Umdrehung des Widerscheinspektrums um 360˚ durch die vorangegebene Geschwindigkeit verstellt wird, welche bei jeder Azimutwinkel den Messvorgang ermöglicht, andererseits der bemessene Widerschein mit dem Spektrum des weißen standardisierten Platten so normalisiert wird, dass diese drei veränderlichen Koeffizienten des Widerscheins schließlich nur in ein veränderliches Koeffizient des Widerschein umgewandelt werden.

**Revendications**

**1.** Appareil de mesurage de couleur qu'on utilise pour le mesurage des coordonnées de couleur et des différences de couleurs de tous types de matériels monochrome ou multicolore qui sont sous forme de fil ou de particule, ou des modèles dures monochrome ou multicolore qui sont illuminés avec un lumière d'éclairage sous l'angle de 0˚ +/- 10˚ en obtenant les spectres de réflexion de l'espace qu'on veut mesure;
**caractérisé en ce qu'**on
l'appareil de mesurage de couleur peut être utilisé ou établi sur un modèle fixé dudit véhicule quand on ne peut pas être établi un modèle sur un mobil plateau tournant ou sur un soutien pour quelconque raison et quand on doit faire le mesurage de couleur dans un procès de production, et **en ce qu'**il comprend

- un source de lumière (18) qui illumine le premier miroir (19) avec un angle précédemment définit et qui se situe sur une plateau (24) ;
- ledit premier miroir (19) qui a été établit sur un soutien retournable (23) situant sous ledit plateau (24) et qui a été placé à une position inclinée pour refléter la lumière à un deuxième miroir (20) ;
- ledit deuxième miroir (20) qui a été établit sur un soutien retournable (23) situant sous ledit plateau (24) et qui a été placé à une position inclinée pour refléter la lumière à ledit modèle;
- les lentilles (21) cohérents avec le plateau (24) de façon qu'elles orientent la lumière et le spectre reflété avec l'angle de 45˚ du modèle illuminé aux unités de mesurage de couleur (22) qui mesure ledit spectre et ladite lumière reflété avec l'angle de 0˚ / 45˚;
- un moteur électrique (6) dont laquelle la régularisation de vitesse angulaire de roue faite par les circuits électroniques et dont on constate la situation angulaire de roue par un codeur (7) ; qui permet de tourner les doubles miroirs avec la vitesse de rotation réglable et d'illuminer le modèle durablement des situations azimutales différents successives.

**2.** Appareil selon la revendication 1 **caractérisé en ce que** l'unité de mesurage de couleur (22) est CCD.

**3.** Appareil selon la revendication 1 **caractérisé en ce que** l'unité de mesurage de couleur (22) est CID.

**4.** Appareil selon la revendication 1 **caractérisé en ce que** l'unité de mesurage de couleur (22) est des photos capteurs qui ont un caractère conjonctif.

**5.** Appareil selon la revendication 1 **caractérisé en ce que** l'unité de mesurage de couleur (22) est des photodiodes ou séries de photodiodes qui marchent avec la méthode de fonctionnement photo voltaïque et photoconducteur.

**6.** Appareil selon la revendication 1 **caractérisé en ce que** l'appareil s'associe avec un processeur digital qui entraîne préférentiellement avec un ordinateur ou avec un software.

**7.** Appareil selon la revendication 1 **caractérisé en ce que** l'unité de mesurage de couleur (22) est ajustée aux valeurs précises qui assurent le mesurage des périodes de digitaliser et de prendre, d'un nombre entière précis des mesurages et du spectre de réflexion, lors d'un tour de 360˚ ou plusieurs tours complets, dans chaque angle azimutale au moyen d'une vitesse prédéterminée, et **en ce qu'**on le reflet mesuré normalise par le spectre de réflexion du blanc plateau standard, et ainsi les trois coefficients variables de reflet sont convertis conséquemment à un seul coefficient variable de reflet.

**8.** Appareil selon la revendication 1 **caractérisé en ce que** l'unité de mesurage de couleur (22) est obtenue conformément à la période de digitaliser et de prendre prédéfinit, **en ce que** l'appareil assure d'un nombre entière précis des mesurages et du spectre de réflexion, lors d'un tour de 360˚ ou plusieurs tours complets, dans chaque angle azimutale, et **en ce qu'**on le reflet mesuré normalise par le spectre de réflexion du blanc plateau standard, et ainsi les trois coefficients variables de reflet sont convertis conséquemment à un seul coefficient variable de reflet.

Figure 1

0°/45°

Figure 2a

45°/0°

Figure 2b

D/8°

Figure 2c

8°/D

Figure 2d

Y

$\varphi = 270°$

$\varphi = 180°$

$\vec{n}$

$\theta = 45°$

$\varphi = 90°$

0

$\Lambda$

$\varphi = 0°$

X

$\varphi$

Figure 3

9

10

6

**Figure 4a**

10

11

6

7

**Figure 4b**

**Figure 5a**

**Figure 5b**

Figure 6a

Figure 6b

Figure 7